(19) Europäisches Patentamt
European Patent Office
Office européen des brevets

(11) Veröffentlichungsnummer: **0 410 366 A2**

(12) **EUROPÄISCHE PATENTANMELDUNG**

(21) Anmeldenummer: **90114134.1**

(51) Int. Cl.[5]: **C07H 15/252**, A61K 31/71

(22) Anmeldetag: **24.07.90**

(30) Priorität: **26.07.89 DE 3924655**

(43) Veröffentlichungstag der Anmeldung:
**30.01.91 Patentblatt 91/05**

(84) Benannte Vertragsstaaten:
**AT BE CH DE DK ES FR GB GR IT LI LU NL SE**

(71) Anmelder: **BEHRINGWERKE Aktiengesellschaft**
**Postfach 1140**
**D-3550 Marburg 1(DE)**

(72) Erfinder: **Kolar, Cenek, Dr.**
**Deutschhausstrasse 20**
**D-3550 Marburg(DE)**
Erfinder: **Dehmel, Konrad**
**Blumengarten 11**
**D-3550 Marburg(DE)**
Erfinder: **Hoffmann, Dieter, Dr.**
**Feuerdornweg 12**
**D-3550 Marburg(DE)**
Erfinder: **Kraemer, Hans Peter, Dr.**
**Birkenweg 16**
**D-3550 Marburg(DE)**
Erfinder: **Gronau, Thomas, Dr.**
**Im Stetefeld 8**
**D-3551 Lahntal(DE)**

(74) Vertreter: **Klein, Otto, Dr. et al**
**Hoechst AG Zentrale Patentabteilung**
**Postfach 80 03 20**
**D-6230 Frankfurt am Main 80(DE)**

(54) **Anthracyclin-Derivate.**

(57) Es werden zytostatisch wirksame Anthracyclin-Derivate, die der Formel I

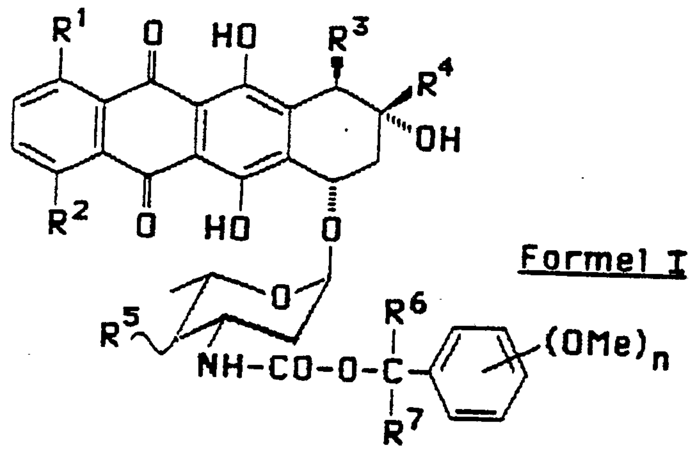

Formel I

entsprechen, worin die Reste folgende Bedeutung haben:
$R^1$ H oder OH,
$R^2$ H, OH oder OMe,
$R^3$ H, OH oder COOMe,
$R^4$ COMe, $COCH_2OH$, CH(OH)Me, $CH(OH)CH_2OH$ oder $C_1$-$C_2$-Alkyl,

EP 0 410 366 A2

Xerox Copy Centre

$R^5$ H, OH, O-Tetrahydropyranyl oder O-Acetyl,
$R^6$ und $R^7$ $C_1$-$C_4$-Alkyl und
n = 1, 2 und/oder 3,
ein Verfahren zur ihrer Herstellung und deren galenischer Zubereitung sowie deren Anwendung für die Behandlung von Krebserkrankungen beschrieben.

## ANTHRACYCLIN-DERIVATE

Die vorliegende Erfindung bezieht sich auf Anthracyclin-Derivate, besonders auf N-3′-Arylalkyloxycarbonylanthracycline, Verfahren zu deren Herstellung sowie deren Anwendung für die Behandlung von Krebserkrankungen.

Die Substanzklasse der Anthracycline ist in der Fachliteratur eingehend beschrieben. Doxorubicin und sein 14-Desoxy-Analogon Daunorubicin sind hier als die erfolgreichsten Vertreter dieser Substanzklasse genannt, die in der Klinik zur Behandlung einer großen Anzahl von festen Tumoren und Leukämien eingesetzt werden. Weitere Analoga, die sowohl in dem Aglyconteil als auch in der Kohlehydrat-Einheit modifiziert wurden, sind kürzlich in die Klinik eingeführt worden oder befinden sich in der klinischen Prüfung.

Es wurde eine Vielzahl von chemischen oder mikrobiellen Verfahren zur Herstellung von Anthracyclinen entwickelt. Hierbei erwiesen sich die Verbindungen, die in der 7-Position des Anthracyclinon-Aglycons eine Kohlehydrat-Einheit des Daunosamin-Typs enthalten, als besonders wirksam. Aufgrund der Struktur-Wirkungsbeziehung der Anthracycline ist bekannt, daß vor allem die 3′-Amino-oder 3′-Mono- oder Dialkylamino-Derivate zytostatisch wirksam sind.

Die 3′-N-Acyl-anthracycline sind meist erheblich weniger wirksam oder sie entfalten ihre zytostatische Wirksamkeit nur gegenüber einer engen Auswahl von Tumorzellen (US 4,035,566).

Überraschenderweise hat sich bei der Untersuchung von 3′-N-(3′,5′-Dimethoxy-α,α-dimethyl-benzyloxycarbonyl)-4′-O-tetrahydropyranyl-Doxorubicin auf dessen zytostatische Wirksamkeit in einem "in vivo" L 1210-Testmodell ergeben, daß die Verbindung erheblich bessere Wirksamkeit als Doxorubicin oder Tetrahydropyranyl-Doxorubicin aufweist. Bei dieser Verbindung hätte man aufgrund des Vorliegens einer Amid-Funktion am C-3′-Atom keine Wirksamkeit erwartet.

Aufbauend auf diesen Erkenntnissen hat es sich die vorliegende Erfindung zur Aufgabe gestellt, ausgehend von Anthracyclinen, die eine Kohlehydrat-Einheit des Daunosamin-Typs enthalten, 3′-N-Arylalkyl-oxycarbonyl-Derivate herzustellen sowie geeignete galenische Zubereitungen für diese neuen Verbindungen zu finden, die ein verbessertes Wirkungsspektrum haben und als tumortherapeutische Mittel angewandt werden können.

Gelöst wird diese Aufgabe mit zytostatisch wirksamen Anthracyclin-Derivaten, die der Formel I

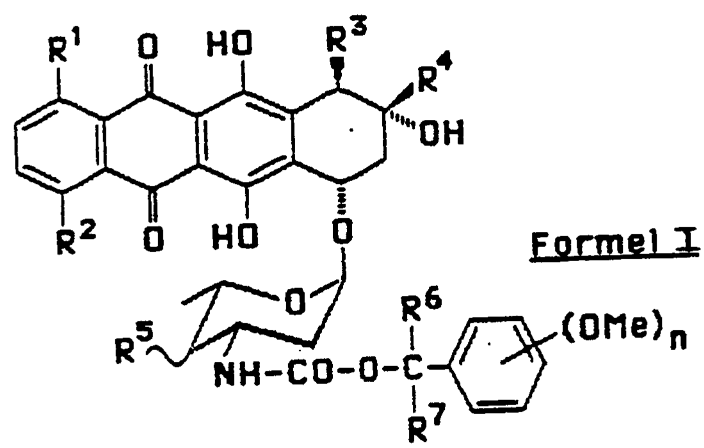

Formel I

entsprechen, worin die Reste folgende Bedeutung haben:

$R^1$ H oder OH,
$R^2$ H, OH oder OMe,
$R^3$ H, OH oder COOMe,
$R^4$ COMe, $COCH_2OH$, CH(OH)Me, $CH(OH)CH_2OH$ oder $C_1$-$C_2$-Alkyl,
$R^5$ H, OH, O-Tetrahydropyranyl oder O-Acetyl,
$R^6$ und $R^7$ $C_1$-$C_4$-Alkyl und
n = 1, 2 und/oder 3.

Bevorzugt sind Verbindungen der Formel I, worin
$R^1$ H oder OH,

$R^2$ H, OH oder OMe,
$R^3$ H, OH oder COOMe,
$R^4$ COMe, $COCH_2OH$ oder Ethyl,
$R^5$ H, OH oder O-Tetrahydropyranyl,
$R^6$ oder $R^7$ Me und
n = 2 sind.

Die Verbindung der Formel I wurde aufgrund ihrer starken Lipophilie mittels eines oder mehrerer Lösungsmittelvermittler in eine physiologische Darreichungsform übergeführt.

Gegenstand der Erfindung ist weiterhin ein Verfahren zur Herstellung einer der Verbindungen der Formel I, dadurch gekennzeichnet, daß man eine Anthracyclin-Verbindung der Formel II

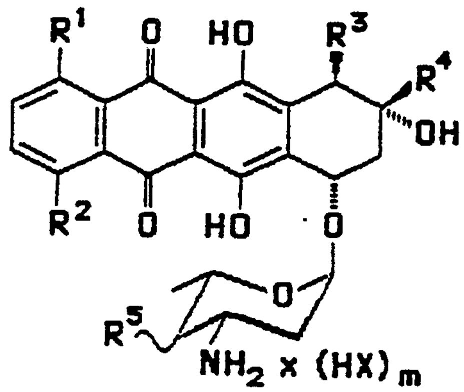

Formel II

worin die Reste
$R^1$ H oder OH,
$R^2$ H, OH oder OMe,
$R^3$ H, OH oder COOMe,
$R^4$ COMe, $COCH_2OH$, CH(OH)Me, $CH(OH)CH_2OH$ oder $C_1$-$C_2$-Alkyl,
$R^5$ H, OH oder O-Tetrahydropyranyl und
HX eine Säure wie HCl mit m = 0 oder 1 darstellen,
mit einer funktionalisierten Arylalkyloxycarbonyl-Verbindung der Formel III,

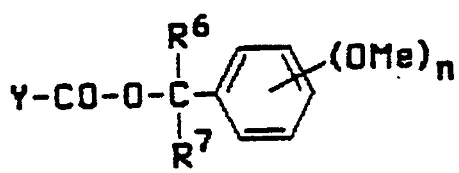

Formel III

worin
$R^6$ und $R^7$ $C_1$-$C_4$-Alkyl,
n = 1,2 und/oder 3 und
Y OH, Cl, $N_3$ oder ein Aktivester sind,
in der Gegenwart einer Base oder eines Carbodiimides in einem organischen Lösungsmittel bei 0° C bis 60° C zu einer Arylalkyloxycarbonylamino-Verbindung der Formel I umsetzt und gegebenenfalls diese Verbindung durch Acylierung oder durch Einführung einer Tetrahydropyranylgruppe zu einer weiteren Verbindung der Formel I modifiziert.

Die Synthese einer Verbindung der Formel I, die von einer Anthracyclin-Verbindung der Formel II, die eine Aminogruppe enthält oder als Ammoniumsalz vorliegt, und einer aktivierten Kohlensäureester-Verbindung der Formel III ausgeht, wird nach den in der Peptidchemie etablierten Verfahren durchgeführt. Hierbei ist es von Vorteil, wenn die Kohlensäureester-Verbindung als Säureazid oder -chlorid vorliegt. Aktivester wie von M. Bodanszki (in The Peptides: Vol. I, Academic Press 1979, S. 105-195) zur Synthese von Peptiden beschrieben, sind ebenfalls anwendbar. Die Verknüpfung der beiden Reaktionspartner der Formel II und der Formel III wird in Gegenwart einer Base wie Triethylamin, Pyridin oder Dimethylaminopyridin in einem organischen Lösungsmittel, wie DMF, Acetonitril, Ethylacetat, Dichlormethan oder Chloroform oder deren Mischungen, gegebenenfalls unter Zusatz von Wasser bei -20° C bis +50° C, bevorzugt bei 0° C bis 25°

C durchgeführt.

Diese entstandenen Verbindungen können gegebenenfalls weiter modifiziert werden, bevorzugt an der OH-4′ Gruppe, durch Einführung einer Acylgruppe wie mit Acetanhydrid in Gegenwart von Pyridin oder durch die Einführung einer Tetrahydropyranylgruppe wie mit Dihydropyran in der Gegenwart von p-Toluolsulfonsäure.

Die Erfindung betrifft weiterhin pharmazeutische Zubereitungen, welche ein Anthracyclin-Derivat der Formel I und ein pharmazeutisches Verdünnungsmittel, einen Lösungsvermittler oder einen Träger enthalten.

Besonders von Vorteil ist die pharmazeutische Zubereitung, bei der eine Verbindung der Formel I in Form einer Emulsion oder einer liposomalen oder micellären Präparation vorliegt. Als Hilfsstoffe zur Herstellung einer pharmazeutisch unbedenklichen Zubereitung können Phospholipide, Cholesterin, Triglyceride oder deren Mischungen verwendet werden. Diese Zubereitungen enthalten eine therapeutisch wirksame Menge der Anthracyclin-Verbindung der Formel I, die bei der Behandlung bestimmter Säugetiertumore ermittelt wird. Als Trägerstoffe eignen sich beispielsweise Albumine, Gelatine-Polymerisate wie Polygelin oder Stärke, die in einer physiologischen Lösung vorliegen.

Die Bestimmung der zytostatischen Wirksamkeit der hier beschriebenen Verbindungen erfolgte in vitro an L 1210-Leukämiezellen in einem Mausmodell oder in vivo an der L 1210 Leukämie, dem B 16 Melanom und dem Lewis Lung Adenokarzinom. Die akute Toxizität der Verbindungen wurde an NMRI-Mäusen ermittelt. Die Methoden sowie Ergebnisse dieser Untersuchung sind in dem experimentellen Teil beschrieben.

Die folgenden Beispiele erläutern die Erfindung.

## Beispiel 1

Herstellung von Anthracyclin-Urethanen des Doxorubicin-Typus

### 7-O-(3-(3′,5′-Dimethoxy-α,α-dimethyl-benzyloxycarbonylamino)-4-O-tetrahydropyranyl-2,3,6-tridesoxy-alpha-L-lyxohexopyranosyl)-adriamycinon (Verbindung 1)

4′-O-Tetrahydropyranyl-doxorubicin (600 mg, 0.95 mmol) wurde in DMF (200 ml) gelöst und mit Triethylamin (0.5 ml) und 3′,5′-Dimethoxy-alpha,alpha-dimethyl-benzyloxycarbonylazid (DDZ-$N_3$) (470 mg, 1.77 mol) unter Lichtausschluß bei 0° C versetzt. Nach 16 h Rühren wurde erneut Triethylamin (0.5 ml) und 3′,5′-Dimethoxy-alpha,alpha-dimethyl-benzyloxycarbonylazid zugegeben. Nach 48 h Rühren wurde der Reaktionsansatz in Vakuum eingedampft. Der Rückstand wurde in Chloroform (250 ml) gelöst und mit 0.1 M Citrat-Puffer (pH 5.5, 100 ml x 2) und dann mit Wasser ausgewaschen. Die organische Phase wurde über Natriumsulfat getrocknet und in Vakuum eingedampft. Das erhaltene Produkt wurde säulenchromatographisch über Kieselgel (150 g) mit 15:1 Dichlormethan-Methanol gereinigt, wobei 760 mg (94 %) der Titelverbindung 1 erhalten wurden.

Schmelzpunkt: 180° - 183° C; $(alpha)_D$ + 139° (c = 0.1 in chloroform) $^1$H NMR (300 MHz, $CDCl_3$); Auswahl: delta 13.89 und 13.16 (2s, 2H, RhOH), 7.99 (dd, 1H, $J_{1,2}$ = 7.7 Hz, $J_{1,3}$ = 0.9 Hz, H-1), 7.76 (dd, 1H, $J_{1,2}$,$J_{2,3}$ = 8.5 Hz, H-2), 7.38 (dd, 1H, H-3), 6.45 (d, 2H, $J_{o,p}$ = 2.2 Hz, DDZ: O,O-Ph-H), 6.29 (t, 1H, DDZ: p-Ph-H), 4.81 (d, 1H, $J_{3',NH}$ = 7.9 Hz, N-H).

### 7-O-(3-(3′,5′-Dimethoxy-α,α-dimethyl-benzyloxycarbonylamino)-2,3,6-tridesoxy-alpha-L-lyxohexopyranosyl)-adriamycinon (Verbindung 2)

Ausgehend von Doxorubicin (300 mg, 0.55 mmol) und DDZ-$N_3$ (190 mg, 0.71 mol) wurde die Titelverbindung 2 nach der Vorschrift zur Synthese von Verbindung 1 hergestellt.

Ausbeute: 3.83 mg (91 %)

Schmelzpunkt: 180-185°C; $(alpha)_D$ + 210° (c = 0.02 in Chloroform) ′H-NMR (200 MHz, $CDCl_3$), delta 13.97 und 13.25 (2s, OH-6 und OH-11), 8.03 (dd, H-1, 7.77 (dd, H-2), 7.39 (dd, H-3), 6.45 (d, DDZ: H-2, H-6), 6.28 (t, DDZ: H-4), 5.09 (d, NH), 4.70 (s, H-14a,b)

7-O-(3-(3 ,5′-Dimethoxy-α,α-dimethyl-benzyloxycarbonylamino)-2,3,6-tridesoxy-alpha-L-arabinohexopyranosyl)-adriamycinon (Verbindung 3 )

Ausgehend von 7-O-(alpha-L-acosaminyl)-adriamycinon (250 mg, 0.46 mmol) und $DDZ\text{-}N_3$ (190 mg, 0.71 mol) wurde die Titelverbindung 3 nach der Vorschrift zur Synthese von Verbindung 1 hergestellt.
Ausbeute: 306 mg (87 %)
Schmelzpunkt: 170-173 $^\circ$ C; $(alpha)_D$ + 326 $^\circ$ (c = 0.1 in Chloroform)

Beispiel 2

Herstellung von Anthracyclin-Urethanen des Daunomycin-Typus

7-O- (3-(3 ,5′-Dimethoxy-α,α-dimethyl-benzyloxycarbonylamino)- 2,3,6-tridesoxy-alpha-L-lyxohexopyranosyl)-daunomycinon (Verbindung 4 )

Daunomycin (500 mg, 0.94 mmol) wurde in 1:1 DMF-Dichlormethan (150 ml) gelöst und mit Triethylamin (0.9 ml) und $DDZ\text{-}N_3$ (380 mg, 1.43 mmol) bei 0 $^\circ$ C versetzt. Die Reaktionsmischung wurde 3 Tage unter Lichtschutz gerührt. Der Ansatz wurde mit Dichlormethan (100 ml) verdünnt und mit 0.1 M Citrat-Puffer (pH 5.5, 100 ml x 2), dann mit Wasser ausgewaschen. Der Rückstand wurde säulenchromatographisch über Kieselgel (120 g) mit 4:1 Dichlormethan-Aceton gereinigt. Es wurden 620 mg (88 %) der Titelverbindung 4 erhalten.
Schmelzpunkt: 144-147 $^\circ$ C, $(alpha)_D$ + 286.6 $^\circ$ (c = 0.03, Chloroform)

Beispiel 3

Herstellung von Anthracyclinen-Urethanen des epsilon-Rhodomycin-Typus

7-O-(3-(3 ,5′-Dimethoxy- , -dimethyl-benzyloxycarbonylamino)-2,3,6-tridesoxy-alpha-L-lyxohexopyranosyl)-epsilon-rhodomycinon (Verbindung 5 )

7-O-(alpha-L-Daunosaminyl)-epsilon-rhodomycinon (120 mg, 0.21 mmol) wurde in 2:1 Ethylacetat-DMF (60 ml) gelöst und mit p-Dimethylaminopyridin (0.2 g) und $DDZ\text{-}N_3$ (80 mg, 0.30 mmol) unter Lichtausschluß bei 0 $^\circ$ C versetzt.

Die Reaktionsmischung wurde 3 Tage gerührt, dann mit Ethylacetat (100 ml) verdünnt und mit 0.1 M Citrat-Puffer (pH 5.5, 60 ml) und Wasser ausgewaschen. Die organische Phase wurde über Natriumsulfat getrocknet und in Vakuum eingedampft. Der Rückstand wurde säulenchromatographisch über Kieselgel (65 g) mit 15:1 Dichlormethan-Methanol gereinigt, wobei 142 mg (87 %) der Titelverbindung 5 erhalten wurden.
Schmelzpunkt: 110 $^\circ$ C; $(alpha)_D$ + 326.5 $^\circ$ (c = 0.02 in Chloroform)

7-O-(4-Desoxy-3-N-(3 ,5′-dimethoxy-α,α-dimethyl-benzyloxycarbonyl)-alpha-L-daunosaminyl)-epsilon-rhodomycinon (Verbindung 6 )

7-O-(4-Desoxy-alpha-L-daunosaminyl)-epsilon-rhodomycinon (400 mg, 0.74 mmol) wurde in DMF (100 ml) gelöst und mit $DDZ\text{-}N_3$ (400 mg, 1.51 mmol) und Triethylamin (0.4 ml) bei 0 $^\circ$ C versetzt. Der Reaktionsansatz wurde 3 Tage bei Raumtemperatur gerührt und, wie üblich, aufgearbeitet. Es wurden 519 mg (92 %) der Titelverbindung 6 erhalten. Schmelzpunkt: 127-130 $^\circ$ C, $(alpha)_D$ + 273 $^\circ$ (c = 0.1, Chloroform)

Beispiel 4

Herstellung von Anthracyclin-Urethanen des epsilon-Isorhodomycin-Typus

7-O-(3-(3,5-Dimethoxy-alpha,alpha-dimethyl-benzyloxycarbonylamino)-2,3,6-tridesoxy-alpha-L-arabinohexopyranosyl)-epsilon-isorhodomycinon (Verbindung 7 )

7-O-(alpha-L-Acosaminyl)-epsilon-isorhodomycinon (250 mg, 0.43 mmol) wurde in DMF (25 ml) gelöst und mit Triethylamin (0.5 ml) und DDZ-$N_3$ (160 mg, 0.60 mmol) unter Lichtausschluß bei Raumtemperatur versetzt. Der Reaktionsansatz wurde 18 h gerührt, dann mit Chloroform (70 ml) verdünnt und mit Wasser (35 ml x 3) ausgewaschen. Die organische Phase wurde über Natriumsulfat getrocknet und in Vakuum eingedampft. Der Rückstand wurde säulenchromatographisch über Kieselgel (160 g) mit 6:1 Dichlormethan-Aceton gereinigt, wobei die Titelverbindung 7 in 267 mg (78 %) Ausbeute erhalten wurde. Schmelzpunkt: 165-168° C; $(alpha)_D$ + 312° (c = 0.025 in Chloroform)

## Beispiel 5

Herstellung von Anthracyclin-Urethanen des β-Rhodomycin-Typus

7-O-(3-(3,5-Dimethoxy-alpha,alpha-dimethyl-benzyloxycarbonylamino)-2,3,6-tridesoxy-alpha-L-lyxohexopyranosyl)-β-rhodomycinon (Verbindung 8 )

7-O-(alpha-L-Daunosaminyl)-β-rhodomycinon (200 mg, 0.38 mmol) wurde in 2:1 Dichlormethan-DMF (50 ml) gelöst und mit Triethylamin (0.21 ml) und DDZ-$N_3$ (200 mg, 0.75 mmol) bei Raumtemperatur versetzt. Nach 24 h wurde der Ansatz mit Dichlormethan (30 ml) verdünnt und mit Wasser (30 ml x 2) ausgewaschen. Die organische Phase wurde über Natriumsulfat getrocknet und in Vakuum eingedampft. Der Rückstand wurde säulenchromatographisch über Kieselgel (140 g) mit 15:1 Dichlormethan-Methanol gereinigt, wobei 232 mg (83 %) der Titelverbindung erhalten wurden.
Schmelzpunkt: 170-173° C; $(alpha)_D$ + 207° (c = 0.1 in Chloroform)

7-O-(4-Desoxy-3-N-(3,5-Dimethoxy-alpha,alpha-dimethylbenzyloxycarbonyl)-alpha-L-daunosaminyl)-β-rhodomycinon (Verbindung 9 )

Ausgehend von 7-O-(4-Desoxy-alpha-L-daunosaminyl)-β-rhodomycinon (120 mg, 0.24 mmol) und DDZ-$N_3$ (70 mg, 0.26 mmol) in der Gegenwart von Triethylamin (1.0 mol) wurde die Titelverbindung nach dem Verfahren zur Herstellung von Verbindung 8 synthetisiert.
Ausbeute: 131 mg (76 %) Schmelzpunkt: 127-130° C; $(alpha)_D$ + 273° (c = 0.1 in Chloroform)

## Beispiel 6

Herstellung von liposomalen Präparationen

Allgemeine Vorschrift:

50 mg DDZ-Anthracyclin, 350 mg Dipalmitoyl-Lecithin, 50 mg Cholesterin wurden in 100 ml Ethanol-Ether 1:1 gelöst und mit 50 ml Wasser intensiv verrührt. Die Mischung wurde in Vakuum bis auf ein Volumen von ca. 40 ml eingeengt und dann lyophilisiert. Der Rückstand wurde in 50 ml wäßriger Kochsalzlösung (9 g NaCl/l Wasser) aufgenommen, und die Mischung wurde 30 min mit Ultraschall behandelt. Der mit einem Coulter Counter TA II ermittelte Durchmesser der Liposomen lag bei 0.5 bis 3 µm.

Verbindung 1 in Liposomen:
Nach der oben beschriebenen Allgemeinen Vorschrift wurden 50 mg Verbindung 1 in Liposomen inkorporiert. Konzentration: 1 g Verbindung 1 pro 1 ml Liposomen-Suspension.
Durchmesser der Liposomen: 1.5-2.0 µm

Verbindung 4 in Liposomen:
Nach der oben beschriebenen allgemeinen Vorschrift wurden 100 mg Verbindung 4 in Liposomen inkorporiert. Konzentration: 1 mg Verbindung 4 pro 1 ml Liposomen-Suspension.
Durchmesser der Liposomen: 1.2-1.8 µm

In gleicher Weise wurde die Verbindung 9 in Liposomen zubereitet.

## Beispiel 7

Herstellung von Lipid-Suspensionen

Allgemeine Vorschrift:

100 ml Lipofundin ® MCT 20 % (Fa. B. Braun Melsungen AG, BRD) wurden lyophilisiert, wobei 23.5 g Trockensubstanz erhalten wurden. Dieser Rückstand und 100 mg Anthracyclin-Verbindung wurden in 100 ml Ethanol-Ether 1:1 intensiv verrührt, dann mit 50 ml Wasser verdünnt, verrührt und bis auf ein Volumen von ca. 40 ml eingeengt. Die verbleibende Suspension wurde lyophilisiert. Der trockene Rückstand wurde mit 100 ml Wasser aufgenommen und im Ultraschallbad 30 min behandelt.

Der mit Nanosizer ermittelte mittlere Durchmesser der Lipidpartikel lag bei ca. 0.5 µm.

Verbindung 1 in Lipidsuspension:
Nach der oben genannten Vorschrift wurden 100 mg Verbindung 1 pro 100 ml Mischung suspendiert.
Durchmesser der Lipidpartikel: 0.4-0.8 µm

In gleicher Weise wurden die Verbindungen 2, 3, 4, 5, 6, 7, 8 und 9 in eine Lipid-Suspension überführt.

## Beispiel 8

Zytotoxizität gegen L 1210 Leukämiezellen (Stem cell assay)

Experimentelles Vorgehen:

Der Test wurde entsprechend der von Hamburger und Salmon beschriebenen Methodik mit den unten beschriebenen Modifikationen durchgeführt.

Konditioniertes Medium wurde durch McCoy 5A Medium ersetzt. Als Folge der hohen Klonierungsrate der L 1210 Leukämiezellen in Soft Agar wurde die Anzahl an Tumorzellen pro Platte auf 5 x $10^2$ reduziert.

Die Zellen wurden mit unterschiedlichen Konzentrationen der Testsubstanz für 1 h bei 37° C inkubiert. Anschließend wurden die Zellen zweimal mit McCoy 5A Medium gewaschen und anschließend in einem Zwei-Schichten-Agar System als obere Schicht entsprechend der Methode von Hamburger und Salmon ausplatiert.

Zusätzliche Parallelexperimente wurden unter Verwendung einer kontinuierlichen Inkubationszeit durchgeführt, wobei unterschiedliche Konzentrationen der Testsubstanz der oberen Agarschicht vor Ausplatieren der Zellen zugemischt wurden.

Die Platten wurden in einem Brutschrank mit 5 % $CO_2$, 20 % 02 und 95 % relativer Luftfeuchte für 5-7 Tage inkubiert. Nach dieser Zeit wurden Kolonien mit einem Durchmesser von 60 µm mit Hilfe eines Invertoskopes gezählt.

Die Ergebnisse wurden angegeben als prozentualer Anteil der Kolonien in behandelten versus unbehandelten Gruppen. Der Variationskoeffizient bei wiederholten Experimenten war kleiner als 15 %.

Resultate:

Die getesteten 3'-N-DDZ-Anthracycline (1, 2, 3, 4 und 8) sind in der Tabelle 1 enthalten.

Aus der Dosis-Wirkungskurve wurde die $IC_{50}$ für die kontinuierliche und einstündige Inkubation bestimmt (Tab. 1).

Die in der Tabelle zusammengefaßten Zahlen belegen, daß die meisten Substanzen eine sehr hohe Zytotoxizität ($IC_{50}$ kleiner als 0.1 µg/ml) in unterschiedlichen in vitro-Testsystemen gegenüber menschlichen und tierischen Tumorzellen aufweisen.

Beispiel 9

Proliferationstest (MTT-Reduktion)

L 1210, A 549 oder HT 29 in exponentieller Wachstumsphase werden in einer Zelldichte von 5 x $10^3$ Zellen/ml in RPMI 1640 in einer 96 Well Mikrotiterplatte für 72 h mit unterschiedlichen Konzentrationen der Testsubstanz bei 37° C, 5 % $CO_2$ und 95 % relativer Luftfeuchte inkubiert. Kontrollexperimente erhalten anstelle der Testsubstanz lediglich Wachstumsmedium. Für jede Testsubstanz sowie für die Kontrolle werden 4fach Bestimmungen angesetzt. Nach 65 h Inkubation werden 50 µl einer MTT-Lösung (2.5 mg/ml in Phosphat-gepuffertem Kochsalz) zugegeben. In Gegenwart lebender Zellen wird MTT zu einem dunkelroten unlöslichen Formazanfarbstoff reduziert. Diese Reaktion ist nach 7 h (L 1210 Zellen) bzw. nach 24 h (A 549, HT 29 Zellen) beendet, und das überstehende Medium wird sorgfältig abgesaugt. Der unlösliche Farbstoff wird durch Zugabe von 100 µl DMSO aufgelöst und die Extinktion der so entstehenden Lösung anschließend für jedes Well in einem Multiscan Photometer 340 CC der Fa. Flow bei einer Wellenlänge von 492 nm vermessen.

Aus dem Verhältnis der Extinktionen behandelter und unbehandelter Zellen ergibt sich eine Dosis-Wirkungskurve, aus der die Konzentration, die gerade 50 % der Zellen ($IC_{50}$) abtötet, abgelesen werden kann. Für wiederholte Untersuchungen beträgt der Variationskoeffizient weniger als 15 % (Tabelle 1).

Tabelle 1

| Verbindung | $IC_{50}$ (µg/ml) | | | |
|---|---|---|---|---|
| | SCA | | MTT | |
| | L 1210 cont/1 h | L1210 | A 549 | HT 29 |
| 1 * | - / 5.3 | 0.027 | 0.023 | 0.04 |
| 1 (in Liposomen) | 0.01/0.64 | 0.01 | 0.03 | 0.02 |
| 1 (in Lipofundin) | - / 3.4 | 0.045 | 0.013 | 0.061 |
| 2 * | - / 6.4 | 0.11 | 0.11 | 0.13 |
| 2 (in Lipofundin) | - / 4.5 | 0.14 | 0.23 | 0.3 |
| 3 * | - / - | 0.02 | 0.04 | 0.11 |
| 3 (in Lipofundin) | - / - | 0.02 | 0.04 | 0.11 |
| 4 * | - / 4.5 | 0.14 | 0.13 | 0.13 |
| 8 * | - / 0.47 | 0.004 | <0.004 | <0.004 |
| 8 (in Lipofundin) | - / 0.32 | <0.004 | <0.004 | 0.0062 |
| Pirarubicin | 0.004/0.01 | 0.004 | 0.15 | 0.016 |

* Präparat wurde wie folgt gelöst in DMSO, Tween 80, Glucose wäßriger Pufferlösung - Konzentration 1 mg Präparat pro 1 ml der Lösung.

Beispiel 10

Ermittlung der akuten Toxizität

Zur Ermittlung der akuten Toxizität werden BDF1-Mäusen am Tag 0 unterschiedliche Dosen der Testsubstanz, gelöst in 0.5 ml 5%iger Glucose-Lösung, intraperitoneal inji ziert. Kontrollgruppen erhalten lediglich 0.5 ml 5%ige Glucose-Lösung. Pro Konzentration der Testsubstanz werden 5 Mäuse verwendet. Am Tag 14 wird die Zahl der überlebenden Mäuse ermittelt und daraus nach der Litchfield Wilcoxon Methode die LD5, LD50 und LD95 ermittelt. Die Toxizität (LD50, mg/kg) der hier beschriebenen Verbindungen im Vergleich zu Adriamycin wurde ermittelt.

Beispiel 11

In vivo Wirksamkeit der erfindungsgemäßen Anthracycline gegen L 1210 Leukämie der Maus (Tabelle 2)

Methodik:
Ascitesflüssigkeit wird unter sterilen Bedingungen DBA2 Mäusen (weiblich, 18-20 g) 7 Tage nach Implantation entnommen. Der Ascites wird dreimal mit PBS gewaschen, gezählt und auf eine Zellzahl von $10^4$ in 0.2 ml PBS eingestellt.

$10^4$ Zellen, suspendiert in 0.2 ml PBS, werden anschließend DBF1 Mäusen (weiblich, 18-20 g) intravenös injiziert. 6 Tiere pro Gruppe werden für jede Substanzkonzentration bzw. als Kontrolle eingesetzt.

Ermittlung der antitumoralen Wirksamkeit:

a) Die Tiere werden am Tag 1 und 5 nach Injektion der Testsubstanz gewogen. Gewichtsverlust von mehr als 20 % am Tag 5 wird als Indikator einer toxischen Substanzwirkung angesehen.
b) Am Ende des Experimentes (Tod aller Tiere oder überlebende Tiere am Tag 60) wird die mittlere Überlebenszeit der Tiere in den jeweiligen Gruppen bestimmt, sofern am Tag 5 des Experimentes mindestens 65 % der Tiere noch gelebt hatten. Die mittlere Überlebenszeit wird ausschließlich für im Verlaufe des Experimentes sterbende Tiere bestimmt. Langzeitüberlebende (LTS) werden bei dieser Berechnung nicht berücksichtigt und gesondert aufgeführt.

Aus der mittleren Überlebenszeit ($MST_t$) der behandelten Gruppen sowie der Kontrollgruppen ($MST_c$) wird die antitumorale Wirksamkeit (T/C) für die jeweilige Substanzkonzentration in Prozent der unbehandelten Kontrolle entsprechend der folgenden Formel bestimmt:

$$T/C\ \% = \frac{MST_t}{MST_c} \times 100$$

T/C Werte größer als 125 % werden als Indikator einer signifikanten antitumoralen Wirksamkeit der Testsubstanz angesehen. Die Dosis, die den größten antitumoralen Effekt (optimale Dosierung) zeigte, wurde ermittelt. Tiere, die am Tag 60 des Experimentes noch leben, werden als Longterm-Survivors getrennt aufgeführt.

Tabelle 2

| Substanz | Schedule | L 1210 | Effektivität | | |
|---|---|---|---|---|---|
| | | opt. Dosis (mg/kg/inj) | T/C % | LTS | toxisch |
| Verbind. 1 | 2xiv/iv Q3D | 20 | 435 | 5/6 | 1/6 |
| Pirarubicin (4′-O-THP-Doxorubicin) | 3xiv/iv Q3D | 4.7 | 178 | - | - |

**Ansprüche**

1. Zytostatisch wirksame Anthracyclin-Derivate, die der Formel I

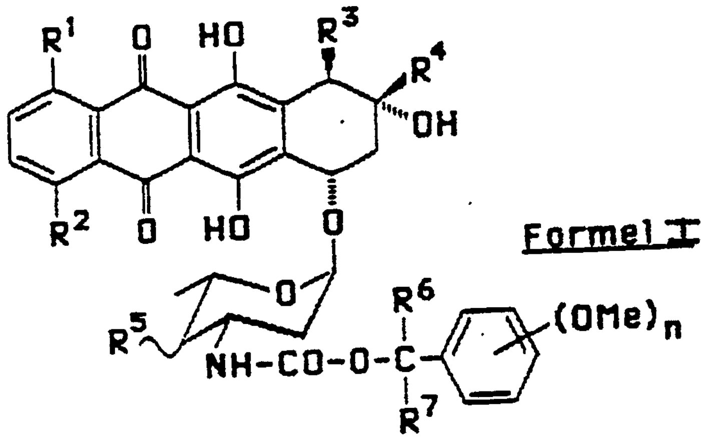

Formel I

entsprechen, worin die Reste folgende Bedeutung haben:
$R^1$ H oder OH,
$R^2$ H, OH oder OMe,
$R^3$ H, OH oder COOMe,
$R^4$ COMe, $COCH_2OH$, CH(OH)Me, $CH(OH)CH_2OH$ oder $C_1$-$C_2$-Alkyl,
$R^5$ H, OH, O-Tetrahydropyranyl oder O-Acetyl,
$R^6$ und $R^7$ C -$C_4$-Alkyl und
n = 1, 2 oder 3.

2. Verbindungen nach Anspruch 1, worin
$R^1$ H oder OH,
$R^2$ H, OH oder OMe,
$R^3$ H, OH oder COOMe,
$R^4$ COMe, $COCH_2OH$ oder Ethyl,
$R^5$ H, OH oder O-Tetrahydropyranyl,
$R^6$ oder R7 Me und
n = 2 sind.

3. Verfahren zur Herstellung einer der Verbindungen nach Anspruch 1, dadurch gekennzeichnet, daß man eine Anthracyclin-Verbindung der Formel II

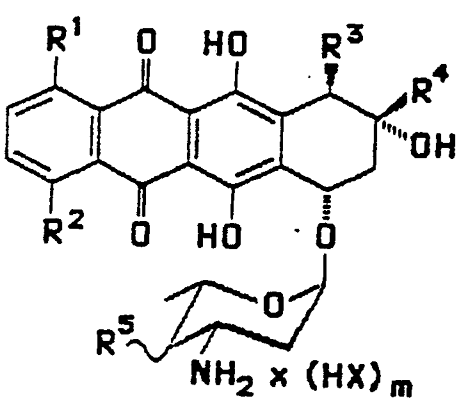

Formel II

worin die Reste
$R^1$ H oder OH,
$R^2$ H, OH oder OMe,
$R^3$ H, OH oder COOMe,
$R^4$ COMe, $COCH_2OH$, CH(OH)Me, $CH(OH)CH_2OH$ oder $C_1$-$C_2$-Alkyl,

$R^5$ H, OH oder O-Tetrahydropyranyl,
HX eine Säure wie HCl mit m = 0 oder 1 darstellen,
mit einer funktionalisierten Arylalkyloxycarbonyl-Verbindung der Formel III,

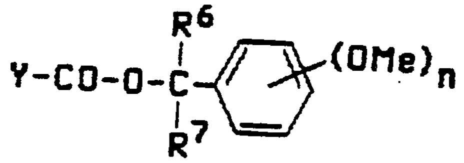

Formel III

worin $R^6$ und $R^7$ $C_1$-$C_4$-Alkyl,
n = 1,2 oder3 und
Y OH, Cl, $N_3$ oder Aktivester sind,
in der Gegenwart einer Base oder eines Carbodiimides in einem organischen Lösungsmittel bei 0° C bis 60° C zu einer Arylalkyloxycarbonylamino-Verbindung der Formel I umsetzt und gegebenenfalls diese Verbindung durch Acylierung oder durch Einführung einer Tetrahydropyranylgruppe zu einer weiteren Verbindung der Formel I modifiziert.

4. Verfahren zur Herstellung einer Verbindung nach Anspruch 3, dadurch gekennzeichnet, daß man eine Anthracyclin-Verbindung der Formel II, wo die Reste wie in Anspruch 3 definiert sind, mit einer funktionalisierten Verbindung der Formel II, worin
$R^6$ und $R^7$ $C_1$-$C_4$-Alkyl,
n = 1, 2 oder 3 und
Y Cl, $N_3$ oder Aktivester sind,
in der Gegenwart einer Base wie Pyridin, Dimethylaminopyridin oder Triethylamin in einem organischen Lösungsmittel wie DMF, Acetonitril, Ethylacetat, Dichlormethan oder Chloroform oder deren Mischungen bei -20° C bis +50° C, bevorzugt bei 0° bis 25° C, zu einer Verbindung der Formel I umsetzt.

5. Verfahren zur Herstellung einer Verbindung der Formel I nach mindestens einem der Ansprüche 3 und 4, dadurch gekennzeichnet, daß man bei einer Verbindung nach Anspruch 1, die eine 4′-OH-Gruppe enthält, diese durch Einführung einer Acylgruppe, wie beispielsweise mit Acetanhydrid in der Gegenwart von Pyridin, oder durch Einführung einer Tetrahydropyranylgruppe mittels Dihydropyran in der Gegenwart einer Säure wie p-Toluolsulfonsäure zu einer weiteren Verbindung der Formel I modifiziert.

6. Verbindung nach Anspruch 1 als Arzneimittel.

7. Pharmazeutische Zubereitung einer Verbindung nach Anspruch 1, dadurch gekennzeichnet, daß sie eine wirksame Menge einer oder mehrerer Verbindungen der Formel I und ein oder mehrere pharmazeutische Verdünnungsmittel, Lösungsvermittler oder Träger enthält.

8. Pharmazeutische Zubereitung nach Anspruch 7, die als Emulsion, liposomale oder micelläre Präparation vorliegt und Phospholipide, Cholesterin, Triglyceride oder deren Mischungen enthält.

9. Pharmazeutische Zubereitung nach Anspruch 7, dadurch gekennzeichnet, daß sie einen oder mehrere Trägerstoffe wie Albumin, bevorzugt humanes Albumin, Gelatine- Polymerisate oder Stärke oder deren Mischungen enthält.

Patentansprüche für folgende Vertragsstaaten:ES, GR

1. Verfahren zur Herstellung eines zytostatisch wirksamen Anthracyclin-Derivates der Formel I,

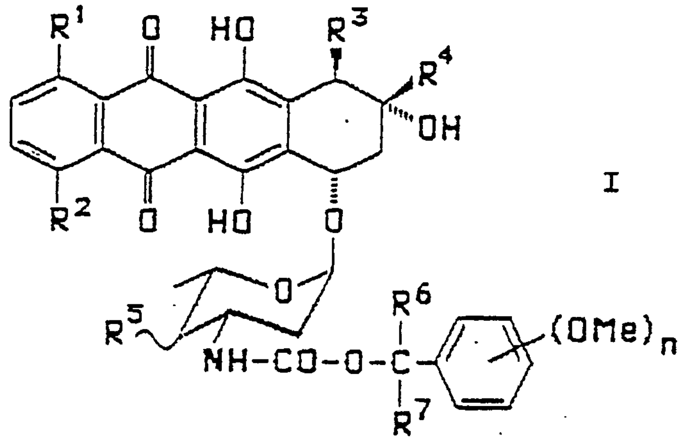

I

worin die Reste folgende Bedeutung haben:
$R^1$ H oder OH,
$R^2$ H, OH oder OMe,
$R^3$ H, OH oder COOMe,
$R^4$ COMe, $COCH_2OH$, CH(OH)Me, $CH(OH)CH_2OH$ oder $C_1$-$C_2$-Alkyl,
$R^5$ H, OH, O-Tetrahydropyranyl oder O-Acetyl,
$R^6$ und $R^7$ $C_1$-$C_4$-Alkyl und
n = 1, 2 oder 3, dadurch gekennzeichnet, daß man eine Anthracyclin-Verbindung der Formel II

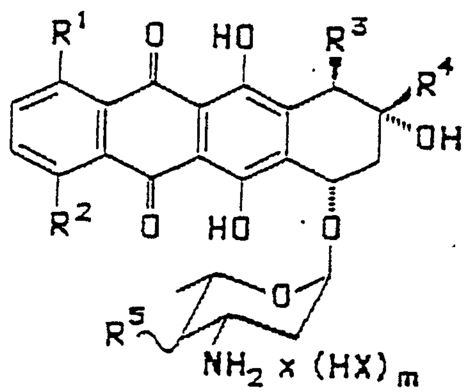

II

worin die Reste
$R^1$ H oder OH,
$R^2$ H, OH oder OMe,
$R^3$ H, OH oder COOMe,
$R^4$ COMe, $COCH_2OH$, CH(OH)Me, $CH(OH)CH_2OH$ oder $C_1$-$C_2$-Alkyl,
$R^5$ H, OH oder O-Tetrahydropyranyl,
HX eine Saure wie HCl mit m = 0 oder 1
darstellen,
mit einer funktionalisierten Arylalkyloxycarbonyl-Verbindung der Formel III,

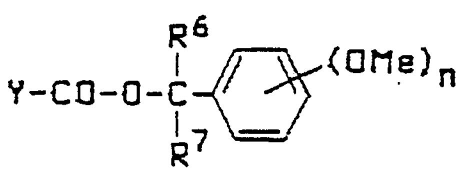

III

worin $R^6$ und $R^7$ $C_1$-$C_4$-Alkyl,
n = 1, 2 oder 3 und

Y OH, Cl, $N_3$ oder Aktivester sind,

in der Gegenwart einer Base oder eines Carbodiimides in einem organischen Losungsmittel bei 0° C bis 60° C zu einer Arylalkyloxycarbonylamino-Verbindung der Formel I umsetzt und gegebenenfalls diese Verbindung durch Acylierung oder durch Einführung einer Tetrahydropyranylgruppe zu einer weiteren Verbindung der Formel I modifiziert.

2. Verfahren zur Herstellung einer Verbindung nach Anspruch 1, dadurch gekennzeichnet, daß man eine Anthracyclin-Verbindung der Formel II, wo die Reste wie in Anspruch 1 definiert sind, mit einer funktionalisierten Verbindung der Formel II, worin

$R^6$ und $R^7$ $C_1$-$C_4$-Alkyl,

n = 1, 2 oder 3 und

Y Cl, $N_3$ oder Aktivester sind,

in der Gegenwart einer Base wie Pyridin, Dimethylaminopyridin oder Triethylamin in einem organischen Losungsmittel wie DMF, Acetonitril, Ethylacetat, Dichlormethan oder Chloroform oder deren Mischungen bei -20° C bis +50° C, bevorzugt bei 0° bis 25° C, zu einer Verbindung der Formel I umsetzt.

3. Verfahren zur Herstellung einer Verbindung nach Anspruch 1, dadurch gekennzeichnet, daß man bei einer Verbindung nach Anspruch 1, die eine 4'-OH-Gruppe enthält, diese durch Einführung einer Acylgruppe, wie beispielsweise mit Acetanhydrid in der Gegenwart von Pyridin, oder durch Einführung einer Tetrahydropyranylgruppe mittels Dihydropyran in der Gegenwart einer Säure wie p-Toluolsulfonsäure zu einer weiteren Verbindung der Formel I modifiziert.

4. Verfahren zur Herstellung einer Verbindung nach Anspruch 2, dadurch gekennzeichnet, daß man bei einer Verbindung nach Anspruch 1, die eine 4'-OH-Gruppe enthält, diese durch Einführung einer Acylgruppe, wie beispielsweise mit Acetanhydrid in der Gegenwart von Pyridin, oder durch Einführung einer Tetrahydropyranylgruppe mittels Dihydropyran in der Gegenwart einer Säure wie p-Toluolsulfonsäure zu einer weiteren Verbindung der Formel I modifiziert.